# EUROPEAN PATENT APPLICATION

(11) **EP 2 065 846 A1**
(43) Date of publication of application: **03.06.2009**
(21) Application number: 08251722.8
(22) Date of filing: 15.05.2008
(51) Int. Cl.: G06Q 10/00

(54) **Patient communication system and method**

(30) Priority: 30.11.2007 US 948103
(71) Applicant: NCR Corporation, Dayton, Ohio 45479 (US)
(72) Inventor: Toleti, Chakravarthy S.K., Windermere, FL 34786 (US); Toleti, Rajesh S.K., Windermere, FL 34786 (US); Vempaty, Nageshwara R., Saratoga, CA 95070 (US)
(74) Representative: MacLeod, Roderick William

(57) **Abstract**

A patient communication system and method is disclosed, which leverages mobile communication devices of patients. The system includes a computer of a healthcare provider for obtaining patient contact information associated with a patient from a patient record, and for sending an electronic message capable of receipt by a mobile communication device of the patient.

## Description

Patient check-in can be a tedious process. When a patient visits a healthcare provider for a scheduled service, the patient typically must check-in with a receptionist. Often, a queue of patients at the front desk leads to a long and uncomfortable wait before the patient is greeted. Standing in line can be cumbersome, especially if the patient is sick.

Patient check-in can be automated using self-service kiosks in healthcare provider offices. Patients may access their appointment records and register for appointments.

Mobile communication devices, such as cellular telephones, personal digital assistants, and handheld computers have become ubiquitous in the last decade. A majority of mobile communication devices support text messaging via the short messaging system (SMS). Mobile communication devices also typically support web access and email.

It would be desirable to provide a system for facilitating patient communication via a mobile communication device, for example, using text messaging, or combinations of text messaging, web access, and email.

A patient communication system and method are provided

A patient communication system and method which leverages mobile communication devices of patients. The system includes a computer of a healthcare provider for obtaining patient contact information associated with a patient from a patient record, and for sending an electronic message capable of receipt by a mobile communication device of the patient.

According to a first aspect of the present invention there is provided a healthcare communication method comprising: obtaining patient contact information associated with a patient from a patient record by a computer controlled by a healthcare provider; and sending an electronic message capable of receipt by a mobile communication device of the patient by the computer.

According to a second aspect of the present invention there is provided a healthcare communication system comprising: a computer of a healthcare provider for obtaining patient contact information associated with a patient from a patient record, and for sending an electronic message to a mobile communication device of the patient.

Embodiments of the present invention will now be described, by way of example, with reference to the accompanying drawings, in which:
Fig. 1 is a block diagram of a healthcare system.
Fig. 2 illustrates an example work flow for checking in a patient via text messaging.
Fig. 3 illustrates an example work flow with more interaction for checking in a patient via text messaging.

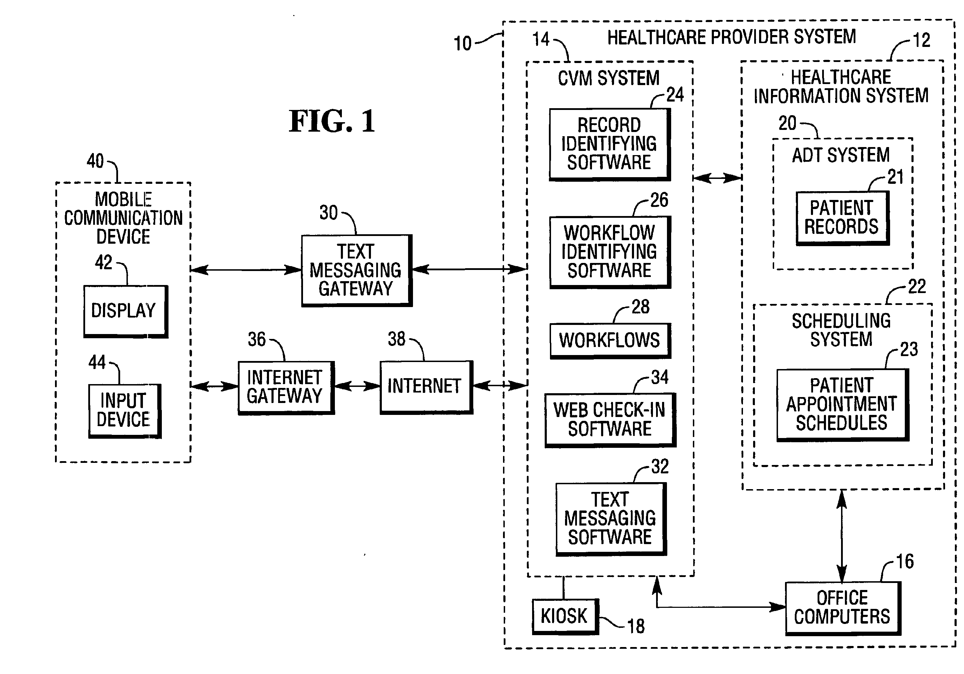

With reference to Fig. 1, healthcare provider system 10 includes healthcare information system 12 and customer value management (CVM) system 14. Healthcare information system 12 stores patient information. Healthcare information system 12 may include admission, discharge, and transfer (ADT) system 20 and scheduling system 22.

ADT system 20 includes patient records 21. Patient records may include mobile phone numbers. Patients provide their mobile phone numbers if they wish to receive appointment reminders via mobile communication devices 40. Pre-registration of mobile communication devices 40 provides a degree of built in security.

Scheduling system 22 includes patient appointment schedules 23.

CVM system 14 executes record identifying software 24 and workflow identifying software 26. Record identifying software 24 attempts to match patient records 21 to patient identifying information presented by patients during checking in. Record identifying software 24 performs the matching process in a way that satisfies a local security policy. The local security policy complies with the Health Insurance Portability and Accountability Act of 1996 (HIPAA).

Workflow identifying software 26 examines the appointment schedule 23 of the patient in scheduling system 22. Workflow 28 may present the patient with requests for information and present questions or guidance based upon the answers. Workflow identifying software 26 examines the purpose of a patient visit and identifies a workflow 28 based on the purpose.

CVM system 14 additionally executes text messaging software 32, web check-in software 34, and email software 38. Text messaging software 32 sends appointment reminders to mobile communication devices 40 of patients and receives patient response from mobile communication devices 40. Reminders and responses are in the form of text messages, which may be communicated through one or more text messaging gateways 30 operated by a cellular communication provider.

Web check-in software 34 processes check-in requests from users of kiosk 18 and mobile communication devices 40. Mobile communication device 40 accesses web check-in software 34 through one ore more internet gateways 36 operated by the cellular communication provider and internet 38.

Email software 38 generates and sends emails to patients, which may be received by mobile communication device 40 through internet 38 and internet gateway 36.

Text messaging software 32 or email software 38 may be used to communicate with a patient. Healthcare provider system 10 may initiate communication for various reasons, such as to remind a patient of an upcoming appointment, inform a patient of lab results, inform a patient of a change in treatment plan such as a new medication or drug regimen, inform a patient of time based events such as check ups or other appointments, or to inform a patient that a provider has not seen the patient for a long time. For purposes of this example embodiment, reminders via text messaging are illustrated.

Text messaging software 32 obtains patient appointment details and a mobile phone number from healthcare information system 12 and sends a text message via text messaging gateway 30 to mobile communication device 40. The text message may include questions, such as whether the patient would like to keep the appointment or change it. The time for sending the text message can be preset or computed based on one or more of the patient's preferences, the patients address, the type of appointment, or other patient data.

The patient sends a text message containing a response. Further interaction with the patient is guided by a registration workflow. The patient and the text messaging software 32 exchange a series of text messages. Specific registration workflows can be customized to the needs of the healthcare provider office and the appointment in question and can be dynamically determined.

Healthcare information system 12, CVM system 14, and mobile communication device 40 include computers with processors and memory for executing programs and storing data, although, healthcare information system 12 and CVM system 14 may be combined into a single computer. More than one healthcare information system 12 may be coupled to CVM system 14.

Healthcare information system 12 and CVM system 14 may be located at a healthcare provider office. If healthcare information system 12 is located externally, CVM system 14 may alternatively access healthcare information system 12 using several methods, including messaging, files, and other communication methods. A common standard for exchanging healthcare data between health care information systems is Health Level 7 (HL7). CVM system 14 can receive patient information and schedule information via messages formatted in HL7 from health care information system 12. Alternatively, it can receive patient information via a flat file in a comma separated value (CSV) format.

Healthcare provider system 10 may additionally include office computers 16 at the front desk and in examination rooms, and one or more check-in kiosks 18 in an office lobby. Healthcare information system 12, customer value management CVM system 14, office computers 16, and kiosk 18 may all be connected via a network.

Mobile communication device 40 may include a portable device, such as a cellular telephone, personal digital assistant, or handheld computer, which is capable of text messaging, accessing web pages, and emailing. These features are often ubiquitous, available in many mobile phones and devices. A patient may be required to pay a special fee to a cellular communication provider to enable these features.

Mobile communication device 40 includes a display 42 for displaying messages and web pages and an input device 44, such as a keypad or touchscreen, for typing or touching response messages or web page entries.

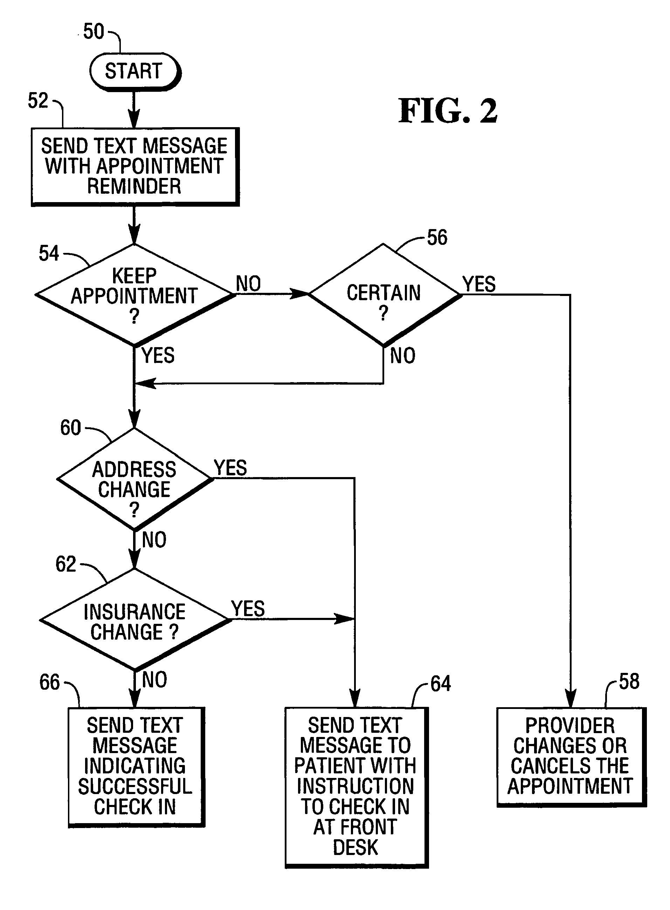

A first example registration workflow is illustrated in Fig. 2, beginning with START 50. In this example workflow, the patient is asked a series of questions. The questions are sent to the patient's mobile communication device 40 by text messaging software 32. The patient replies to each question by sending a reply text message with a "yes" or "no" answer. The workflow and the questions asked are kept simple for the purpose of illustration. More complex workflows are envisioned.

In step 52, text messaging software 32 sends a text message to a patient's mobile communication device 40.

In step 54, mobile communication device 40 displays the message, which includes a question asking the patient whether the patient would like to keep the scheduled appointment. The patient enters a response into mobile communication device 40 and sends the response as a text message. If the patient answers "no", operation continues to step 56. If the patient answers "yes", operation continues to step 60.

In step 56, text messaging software 32 receives the response text message from mobile communication device 40 and sends another text message. Mobile communication device 40 displays the message, which includes a question asking the patient to confirm that the appointment should be canceled or rescheduled. The patient enters a response into mobile communication device 40 and sends the response as a text message. If the patient answers "no", operation continues to step 60. If the patient answers "yes", operation continues to step 58.

In step 58, CVM system 14 initiates a cancellation or change of the appointment in healthcare information system. CVM system 14 may additionally initiate another workflow directed to rescheduling the appointment.

In step 60, text messaging software 32 receives the response text message from steps 54 or 56 and sends another text message. Mobile communication device 40 displays the message, which includes a question asking the patient whether the patient's address has changed. The patient enters a response into mobile communication device 40 and sends the response as a text message. If the patient answers "no", operation continues to step 62. If the patient answers "yes", operation continues to step 64.

In step 62, text messaging software 32 receives the response text message from step 60 and sends another text message. Mobile communication device 40 displays the message, which includes a question asking the patient whether the patient's insurance has changed. The patient enters a response into mobile communication device 40 and sends the response as a text message. If the patient answers "no", operation continues to step 66. If the patient answers "yes", operation continues to step 64.

In step 64, text messaging software 32 receives patient responses from steps 60 and 62 and sends a message. Mobile communication device 40 receives and displays the message, which includes an instruction to the patient to check in at the front desk of the healthcare provider. The receptionist may then assist the patient with updating of patient information. The message may also include directions to the healthcare provider.

As illustrated here, any of the check-in workflows may include a combination of possible channels, including but not limited to, a call center, a front desk, a kiosk, and a portal through many types of communication methods, including text messaging, web page entries, and email.

In step 66, text messaging software 32 receives the response text message from step 62 and sends another text message. Mobile communication device 40 displays the message, which notifies the patient that the patient has successfully checked in.

To augment the check-in experience, for example, with consideration given to customer preferences, text messaging software 32 may instead display a link to a check-in web page under the control of web check-in software 34.

Healthcare provider system 10 may follow up the exchange with a email message to the patient, which the patient may receive on mobile communication device 40

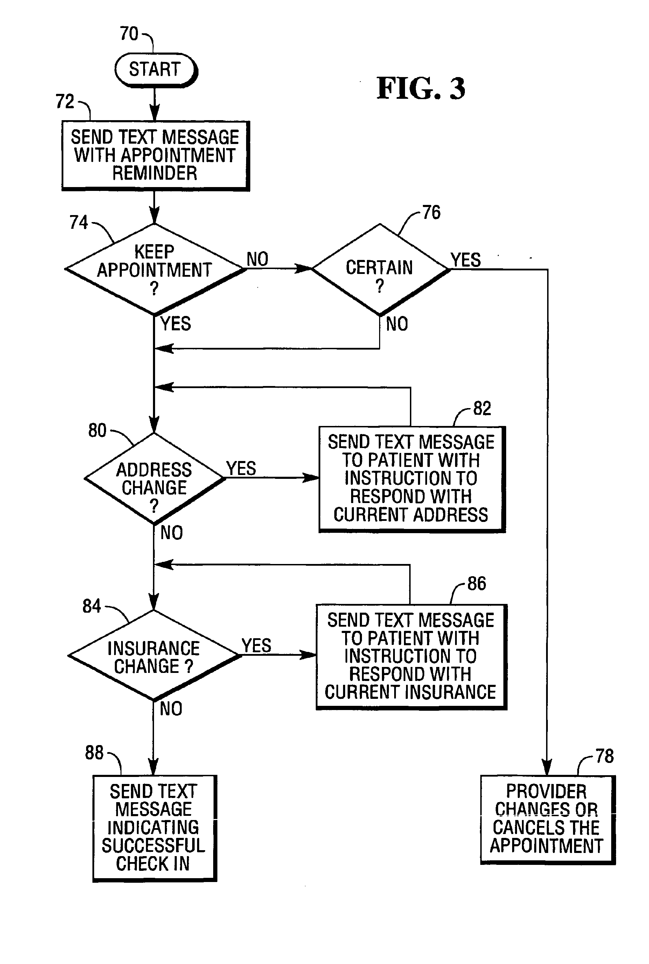

With reference to Fig. 3, another example workflow with more patient interaction begins with START 70. This workflow may be more suitable for mobile communication devices 40 which have large keypads that make text input easier, however, it may still be suitable for patients who are adept with text messaging on their mobile communication devices 40, regardless of device type.

In step 72, text messaging software 32 sends a text message to a patient's mobile communication device 40.

In step 74, mobile communication device 40 displays the message, which includes a question asking the patient whether the patient would like to keep the scheduled appointment. The patient enters a response into mobile communication device 40 and sends the response as a text message. If the patient answers "no", operation continues to step 76. If the patient answers "yes", operation continues to step 80.

In step 76, text messaging software 32 receives the response text message from mobile communication device 40 and sends another text message. Mobile communication device 40 displays the message, which includes a question asking the patient to confirm that the appointment should be canceled or rescheduled. The patient enters a response into mobile communication device 40 and sends the response as a text message. If the patient answers "no", operation continues to step 80. If the patient answers "yes", operation continues to step 78.

In step 78, CVM system 14 initiates a cancellation or change of the appointment in healthcare information system. CVM system 14 may additionally initiate another workflow directed to rescheduling the appointment.

In step 80, text messaging software 32 receives the response text message from steps 74 or 76 and sends another text message. Mobile communication device 40 displays the message, which includes the patient's address of record and a question asking the patient whether the patient's address is correct. The patient enters a response into mobile communication device 40 and sends the response as a text message. If the patient answers "no", operation continues to step 84. If the patient answers "yes", operation continues to step 82.

In step 82, text messaging software 32 receives the response text message from step 80 and sends another text message. Mobile communication device 40 displays the message, which includes an instruction to send a current address. The patient enters a response into mobile communication device 40 and sends the response as a text message. Operation loops back to step 80.

In step 84, text messaging software 32 receives the response text message from step 80 and sends another text message. Mobile communication device 40 displays the message, which includes insurance information of record and a question asking the patient whether the patient's insurance has changed. The patient enters a response into mobile communication device 40 and sends the response as a text message. If the patient answers "no", operation continues to step 88. If the patient answers "yes", operation continues to step 86.

In step 86, text messaging software 32 receives the response text message from step 84 and sends another text message. Mobile communication device 40 displays the message, which includes an instruction to send current insurance information. The patient enters a response into mobile communication device 40 and sends the response as a text message. Operation loops back to step 84.

In step 88, text messaging software 32 receives the response text message from step 84 and sends another text message. Mobile communication device 40 displays the message, which notifies the patient that the patient has successfully checked in. The message may also include directions to the healthcare provider.

As with the workflow of Fig. 2, text messaging software 32 may instead display a link to a check-in web page under the control of web check-in software 34.

Healthcare provider system 10 may follow up the exchange with a email message to the patient, which the patient may receive on mobile communication device 40

Advantageously, patients who check in remotely can avoid waiting in long lines at healthcare provider offices. Patients are engaged before they arrive at appointments, so questions about changes in a patient's address, insurance coverage, and other housekeeping matters may be handled remotely rather than at healthcare provider offices.

The embodiments of Figs. 2 and 3 may be combined or customized to suit a particular patient's mobile communication device 40, local, language, or comfort level in text messaging. In a different embodiment, a patient that has difficulty with text messaging may be differed to other means such as an operator or a front desk receptionist for handling the check-in process. Patient contact preferences may be stored in patient records 21 along with patient contact information.

Although particular reference has been made to certain embodiments, variations and modifications are also envisioned within the scope of the following claims.

## Claims

1. A healthcare communication method comprising:
obtaining patient contact information associated with a patient from a patient record by a computer controlled by a healthcare provider; and
sending an electronic message capable of receipt by a mobile communication device of the patient by the computer.

2. The method of claim 1, further comprising:
obtaining patient schedule information by the computer;
wherein the electronic message notifies the patient of an upcoming appointment.

3. The method of claim 2, wherein the electronic message further includes a question asking the patient whether the patient intends to keep the appointment.

4. The method of claim 3, further comprising:
receiving a check-in electronic message from the mobile communication device.

5. The method of claim 1, further comprising:
receiving a response electronic message from the mobile communication device.

6. The method of claim 1, wherein the electronic message contains a link to a website of the computer for checking in, and wherein the method further comprises processing a check-in request of the patient via the website by the computer.

7. The method of any preceding claim, further comprising:
obtaining patient preferences from the patient record by the computer; and
determining a communication workflow for interacting with the patient based upon the patient preferences.

8. The method of any preceding claim, wherein the patient preferences contain information related to skill of the patient at text messaging.

9. A healthcare communication system comprising:
a computer of a healthcare provider for obtaining patient contact information associated with a patient from a patient record, and for sending an electronic message to a mobile communication device of the patient.

10. The system of claim 9, wherein the computer is also for obtaining patient schedule information, and wherein the electronic message notifies the patient of an upcoming appointment.

11. The system of claim 9, wherein the computer is also for receiving a check-in electronic message from the mobile communication device.

12. The system of claim 10, wherein the computer is also for receiving a response electronic message from the mobile communication device.

13. The system of claim 10, wherein the electronic message contains a link to a website of the computer for checking in, and wherein the computer is also for processing a check-in request of the patient via the website.

14. The system of claim 9, wherein the computer is also for obtaining patient preferences from the patient record, and for determining a communication workflow for interacting with the patient based upon the patient preferences.

15. The system of claim 14, wherein the patient preferences contain information related to skill of the patient at text messaging.
